# EUROPEAN PATENT APPLICATION

(11) **EP 4 597 459 A1**
(43) Date of publication of application: **06.08.2025**
(21) Application number: 22960826.0
(22) Date of filing: 27.09.2022
(51) Int. Cl.: G06V 40/18, G06T 7/00

(54) **INFORMATION PROCESSING SYSTEM, INFORMATION PROCESSING METHOD, AND RECORDING MEDIUM**

(71) Applicant: NEC Corporation, 108-8001 Tokyo (JP)
(72) Inventor: AKIBA, Hirokazu, Tokyo 108-8001 (JP)
(74) Representative: Betten & Resch
(86) International application number: PCT/JP2022/035974
(87) International publication number: WO 2024/069762

(57) **Abstract**

An information processing system includes: an acquisition unit that acquires an eye image including an eye of a target person; a determination unit that determines whether or not the eye image is an image suitable for registration; a detection unit that detects an opening degree of the eye of the target person in the eye image in a case where it is determined that the eye image is not suitable for registration; and an output unit that outputs guide information for encouraging the target person to open the eye with a finger, based on the opening degree of the eye. According to this information processing system, a high-quality eye image can be obtained by encouraging the target person to open the eye.

## Description

### Technical Field

This disclosure relates to technical fields of an information processing system, an information processing method, and a recording medium.

### Background Art

A known system of this type outputs information for improving quality of an image when an image of an eye is captured. For example, Patent Literature 1 discloses that it is determined whether or not an opening degree of an eyelid is greater than or equal to a predetermined value, and that a guidance instruction is given when the opening degree of the eyelid is not sufficient. Patent Literature 2 discloses that a state of the eyelid of an eye to be examined (an open eyelid state) is detected, and that if the state of the eyelid is insufficient, it is disclosed to notify an examiner of the insufficiency.

### Citation List

### Patent Literature

Patent Literature 1: International Publication No. WO2021/245823
Patent Literature 2: JP2009-131591A

### Summary

### Technical Problem

This disclosure aims to improve the techniques/technologies disclosed in Citation List.

### Solution to Problem

An information processing system according to an example aspect of this disclosure includes: an acquisition unit that acquires an eye image including an eye of a target person; a determination unit that determines whether or not the eye image is an image suitable for registration; a detection unit that detects an opening degree of the eye of the target person in the eye image in a case where it is determined that the eye image is not suitable for registration; and an output unit that outputs guide information for encouraging the target person to open the eye with a finger, based on the opening degree of the eye.

An information processing method according to an example aspect of this disclosure includes: acquiring an eye image including an eye of a target person; determining whether or not the eye image is an image suitable for registration; detecting an opening degree of the eye of the target person in the eye image in a case where it is determined that the eye image is not suitable for registration; and outputting guide information for encouraging the target person to open the eye with a finger, based on the opening degree of the eye.

A recording medium according to an example aspect of this disclosure is a recording medium on which a computer program that allows at least one computer to execute an information processing method is recorded, the information processing method including: acquiring an eye image including an eye of a target person; determining whether or not the eye image is an image suitable for registration; detecting an opening degree of the eye of the target person in the eye image in a case where it is determined that the eye image is not suitable for registration; and outputting guide information for encouraging the target person to open the eye with a finger, based on the opening degree of the eye.

### Brief Description of Drawings

[FIG. 1] FIG. 1 is a block diagram illustrating a hardware configuration of an information processing system according to a first example embodiment.
[FIG. 2] FIG. 2 is a block diagram illustrating a functional configuration of the information processing system according to the first example embodiment.
[FIG. 3] FIG. 3 is a flowchart illustrating a flow of operation of the information processing system according to the first example embodiment.
[FIG. 4] FIG. 4 is a plan view illustrating an example of guide information in the information processing system according to the first example embodiment.
[FIG. 5] FIG. 5 is a flowchart illustrating a flow of operation of an information processing system according to a second example embodiment.
[FIG. 6] FIG. 6 is a plan view illustrating an example of the guide information in the information processing system according to the second example embodiment.
[FIG. 7] FIG. 7 is a flowchart illustrating a flow of operation of an information processing system according to a third example embodiment.
[FIG. 8] FIG. 8 is a plan view illustrating an example of the guide information in the information processing system according to the third example embodiment.
[FIG. 9] FIG. 9 is a flowchart illustrating a flow of operation of an information processing system according to a fourth example embodiment.
[FIG. 10] FIG. 10 is a block diagram illustrating a functional configuration of an information processing system according to a fifth example embodiment.
[FIG. 11] FIG. 11 is a flowchart illustrating a flow of operation of the information processing system according to the fifth example embodiment.
[FIG. 12] FIG. 12 is a plan view illustrating an example of the guide information in the information processing system according to the fifth example embodiment.

### Description of Example Embodiments

Hereinafter, an information processing system, an information processing method, and a recording medium will be described with reference to the drawings.

### <First Example Embodiment>

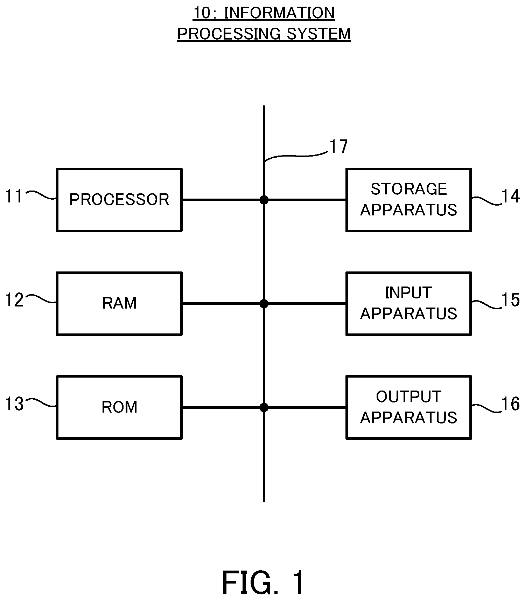

An information processing system according to a first example embodiment will be described with reference to FIG. 1 to FIG. 4.

### (Hardware Configuration)

First, with reference to FIG. 1, a hardware configuration of the information processing system according to the first example embodiment will be described. FIG. 1 is a block diagram illustrating the hardware configuration of the information processing system according to the first example embodiment.

As illustrated in FIG. 1, an information processing system 10 according to the first example embodiment includes a processor 11, a RAM (Random Access Memory) 12, a ROM (Read Only Memory) 13, and a storage apparatus 14. The information processing system 10 may further include an input apparatus 15 and an output apparatus 16. The processor 11, the RAM 12, the ROM 13, the storage apparatus 14, the input apparatus 15, and the output apparatus 16 are connected through a data bus 17.

The processor 11 reads a computer program. For example, the processor 11 is configured to read a computer program stored by at least one of the RAM 12, the ROM 13, and the storage apparatus 14. Alternatively, the processor 11 may read a computer program stored in a computer-readable recording medium, by using a not-illustrated recording medium reading apparatus. The processor 11 may acquire (i.e., may read) a computer program from a not-illustrated apparatus disposed outside the information processing system 10, through a network interface. The processor 11 controls the RAM 12, the storage apparatus 14, the input apparatus 15, and the output apparatus 16 by executing the read computer program. Especially in the present example embodiment, when the processor 11 executes the read computer program, a functional block for outputting guide information when an eye image is captured, is realized or implemented in the processor 11. That is, the processor 11 may function as a controller for executing each control in the information processing system 10.

The processor 11 may be configured as, for example, a CPU (Central Processing Unit), a GPU (Graphics Processing Unit), a FPGA (Field-Programmable Gate Array), a DSP (Demand-Side Platform), an ASIC (Application Specific Integrated Circuit), or a quantum processor. The processor 11 may be one of them, or may use a plurality of them in parallel.

The RAM 12 temporarily stores the computer program to be executed by the processor 11. The RAM 12 temporarily stores data that are temporarily used by the processor 11 when the processor 11executes the computer program. The RAM 12 may be, for example, a D-RAM (Dynamic Random Access Memory) or a SRAM (Static Random Access Memory). Furthermore, another type of volatile memory may also be used instead of the RAM 12.

The ROM 13 stores the computer program to be executed by the processor 11. The ROM 13 may otherwise store fixed data. The ROM 13 may be, for example, a P-ROM (Programmable Read Only Memory) or an EPROM (Erasable Read Only Memory). Furthermore, another type of non-volatile memory may also be used instead of the ROM 13.

The storage apparatus 14 stores data that are stored by the information processing system 10 for a long time. The storage apparatus 14 may operate as a temporary/transitory storage apparatus of the processor 11. The storage apparatus 14 may include, for example, at least one of a hard disk apparatus, a magneto-optical disk apparatus, a SSD (Solid State Drive), and a disk array apparatus.

The input apparatus 15 is an apparatus that receives an input instruction from a user of the information processing system 10. The input apparatus 15 may include, for example, at least one of a keyboard, a mouse, and a touch panel. The input apparatus 15 may be configured as a portable terminal such as a smartphone and a tablet. The input apparatus 15 may be an apparatus that allows audio input/voice input, including a microphone, for example.

The output apparatus 16 is an apparatus that outputs information about the information processing system10 to the outside. For example, the output apparatus 16 may be a display apparatus (e.g., a display) that is configured to display the information about the information processing system 10. The output apparatus 16 may be a speaker or the like that is configured to audio-output the information about the information processing system 10. The output apparatus 16 may be configured as a portable terminal such as a smartphone and a tablet. The output apparatus 16 may be an apparatus that outputs information in a form other than an image. For example, the output apparatus 16 may be a speaker that audio-outputs the information about the information processing system 10.

Although FIG. 1 illustrates the information processing system 10 including a plurality of apparatuses, all or a part of the functions may be realized or implemented in a single apparatus (an information processing apparatus). In such a case, the information processing apparatus may include, for example, only the processor 11, the RAM 12, and the ROM 13. The other components (i.e., the storage apparatus 14, the input apparatus 15, and the output apparatus 16) may be provided in an external apparatus connected to the information processing apparatus. In addition, in the information processing apparatus, a part of an arithmetic function may be realized by an external apparatus (e.g., an external server or cloud, etc.).

### (Functional Configuration)

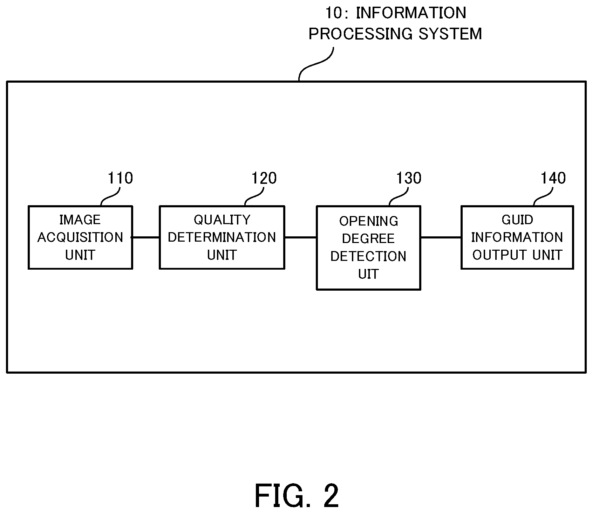

Next, with reference to FIG. 2, a functional configuration of the information processing system 10 according to the first example embodiment will be described. FIG. 2 is a block diagram illustrating the functional configuration of the information processing system according to the first example embodiment.

As illustrated in FIG. 2, the information processing system 10 according to the first example embodiment includes, as components for realizing the functions thereof, an image acquisition unit 110, a quality determination unit 120, an opening degree detection unit 130, and a guide information output unit 140. Each of the image acquisition unit 110, the quality determination unit 120, the opening degree detection unit 130, and the guide information output unit 140 may be a processing block realized or implemented by the processor 11 (see FIG. 1), for example.

The image acquisition unit 110 is configured to acquire an eye image including an eye of a target person. This eye image is registered as an image indicating the eye of the target person. For example, the eye image may be an image registered as a registration image of iris authentication (i.e., an eye image indicating an iris of a registered user). The eye image acquired by the image acquisition unit 110 is configured to be outputted to the quality determination unit 120.

The quality determination unit 120 is configured to determine whether or not the eye image acquired by the image acquisition unit 110 is an image suitable for registration. Whether or not the eye image is suitable for registration may be determined, for example, on the basis of a score indicating quality of the eye image. In this case, the quality determination unit 120 may be configured to determine whether or not the score indicating the quality of the eye image is greater than or equal to a predetermined threshold, for example. A determination result by the quality determination unit 120 is configured to be outputted to the opening degree detection unit 130.

The opening degree detection unit 130 is configured to detect an opening degree of the eye of the target person in the eye image, when it is determined by the quality determination unit 120 that the eye image is not suitable for registration. For example, as the opening degree of the eye, the opening degree detection unit 130 may detect a degree of an eyelid overlapping an iris, or a degree of the iris hidden by the eyelid. Since existing techniques/technologies can be properly adopted to a specific method of detecting the opening degree of the eye, a detailed description of the method will be omitted here. A detection result by the opening degree detection unit 130 is configured to be outputted to the guide information output unit 140.

The guide information output unit 140 is configured to output guide information for encouraging the target person to open the eye with a finger on the basis of the opening degree of the eye detected by the opening degree detection unit 130. For example, the guide information may include information for encouraging the target person to open the eye with the finger. The guide information may be displayed as an image by using a display, for example. Alternatively, the guide information may be audio-outputted by using a speaker. The guide information may be outputted to an apparatus provided around the target person, or may be outputted to a terminal held by the target person, or the like. A specific example of the guide information will be described in detail later.

### (Flow of Operation)

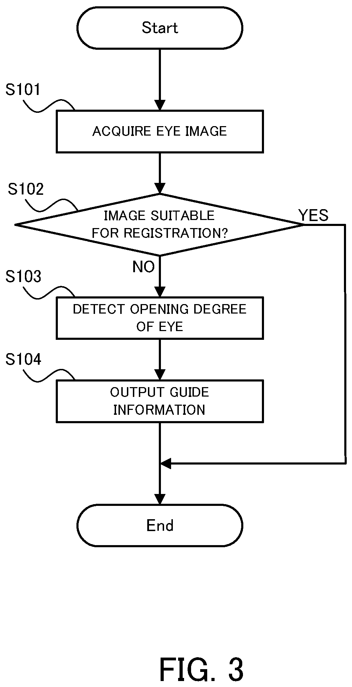

Next, with reference to FIG. 3, a flow of operation by the information processing system 10 according to the first example embodiment will be described. FIG. 3 is a flowchart illustrating the flow of the operation of the information processing system according to the first example embodiment.

As illustrated in FIG. 3, when the operation of the information processing system 10 according to the first example embodiment is started, first, the image acquisition unit 110 acquires the eye image including the eye of the target person (step S101). Then, the quality determination unit 120 determines whether or not the eye image acquired by the image acquisition unit 110 is an image suitable for registration (step S102). When it is determined that the eye image is an image suitable for registration (the step S102: YES), the subsequent processing is omitted and a series of operation steps is ended. In this instance, the information processing system 10 may perform processing of registering the eye image that is determined to be suitable for registration (i.e., processing of storing the acquired image as the eye image of the target person).

On the other hand, when it is determined that the eye image is an image unsuitable for registration (the step S102: NO), the opening detection unit 130 detects the opening degree of the eye of the target person in the eye image (step S103). Then, the guide information output unit 140 outputs the guide information based on the opening degree of the eye detected by the opening degree detection unit 130 (step S104). This makes it possible to encourage the target person to open the eye with the finger.

The image acquisition unit 110 may acquire the eye image of the target person who is opening the eye with the finger after the guide information is outputted. Then, it may perform processing of registering a newly acquired eye image (i.e., an eye image whose quality is improved by opening the eye), as the eye image of the target person.

### (Example of Guide information)

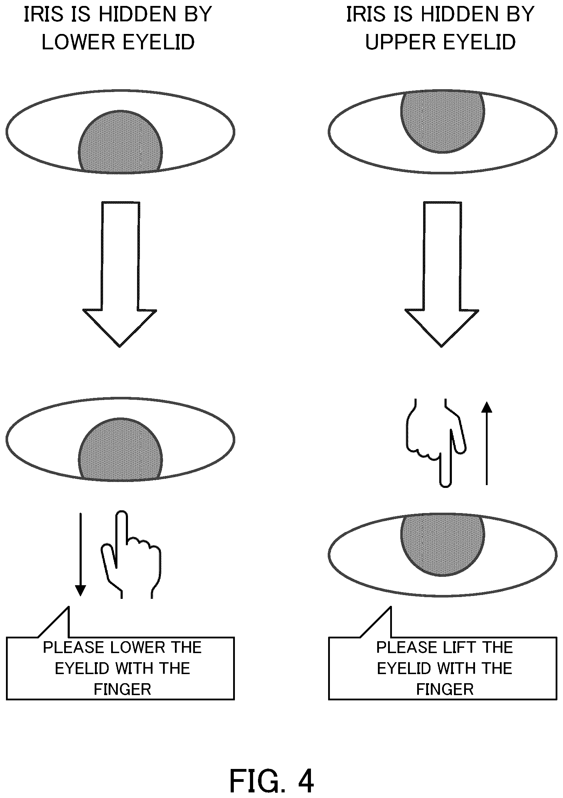

Next, with reference to FIG. 4, a specific example of the guide information in the information processing system 10 according to the first example embodiment will be described. FIG. 4 is a plan view illustrating an example of the guide information in the information processing system according to the first example embodiment.

As illustrated in FIG. 4, the guide information outputted by the information processing system 10 according to the first example embodiment may be information for encouraging an action of lifting or lowering the eyelid overlapping the iris. For example, when a lower eyelid overlaps the iris (i.e., a lower side of the iris is hidden), the guide information may include a message "Please lower the eyelid down with the finger." Furthermore, when an upper eyelid overlaps the iris (i.e., an upper side of the iris is hidden), the guide information may include a message "Please lift the eyelid up with the finger".

The guidance information may include an image of the finger or an arrow (i.e., an arrow indicating a direction of moving the finger) or the like, in order to clearly explain the action to the target person. At this time, the image of the finger or the arrow may be displayed such that it moves. For example, when the action of lowering the eyelid with the finger is encouraged, the image of the finger may move downward, or the arrow may extend downward.

### (Technical Effect)

Next, a technical effect obtained by the information processing system 10 according to the first example embodiment will be described.

As described in FIG. 1 to FIG. 4, in the information processing system 10 according to the first example embodiment, when it is determined that the eye image of the target person is not suitable for registration, the guide information according to the opening degree of the eye of the target person (specifically, information for encouraging the target person to open the eye with the finger) is outputted. In this way, it is possible to encourage the target person to open the eye with the finger. Consequently, it is possible to capture the eye image with the eye sufficiently open, and to acquire a high-quality eye image (e.g., an image clearly showing the entire iris). It is thus possible to improve accuracy of biometric authentication using the registered eye image, for example.

The effect of the present example embodiment is remarkable when the eyelid of the target person is drooping, when the eyelid is swollen with fat, when muscles of the eyelid are weakened, when motor nerves of the brain to open the eyelid are not functioning, or in similar cases. The target person as described above, is not able to sufficiently open the eye, even when the guide information such as "Please open your eye wide" is outputted, and it is therefore hard to acquire an appropriate eye image. According to the information processing system 10 in the present example embodiment, however, the target person can be encouraged to open the eye with the finger, and it is therefore possible to acquire the appropriate eye image even from the target person who has difficulty in opening the eye wide.

### <Second Example Embodiment>

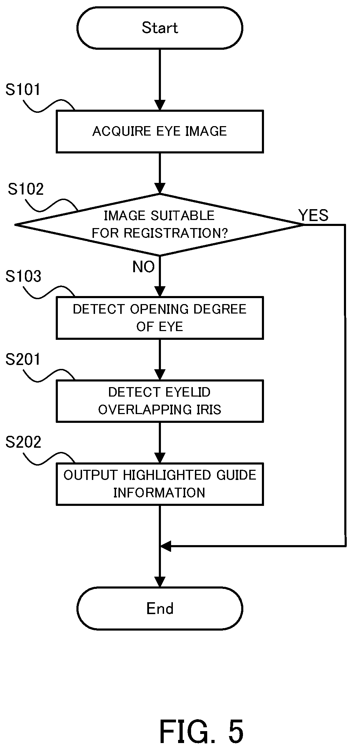

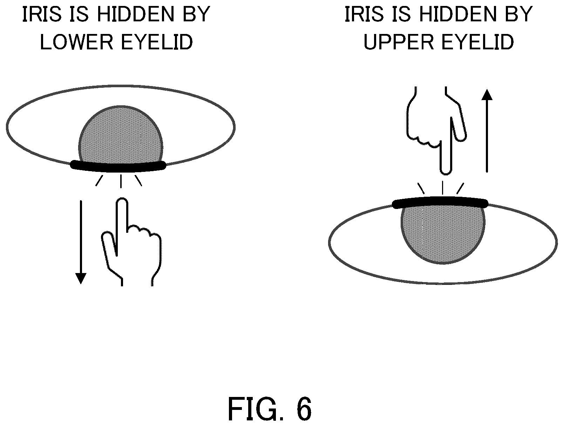

The information processing system 10 according to a second example embodiment will be described with reference to FIG. 5 and FIG. 6. The second example embodiment is partially different from the first example embodiment only in the operation, and may be the same as the first example embodiment in the other parts. For this reason, a part that is different from the first example embodiment will be described in detail below, and a description of the other overlapping parts will be omitted as appropriate.

### (Flow of Operation)

First, with reference to FIG. 5, a flow of operation by the information processing system 10 according to the second example embodiment will be described. FIG. 5 is a flowchart illustrating the flow of the operation of the information processing system according to the second example embodiment. In FIG. 5, the same steps as those illustrated in FIG. 3 carry the same reference numerals.

As illustrated in FIG. 5, when the operation of the information processing system 10 according to the second example embodiment is started, first, the image acquisition unit 110 acquires the eye image including the eye of the target person (step S101). Then, the quality determination unit 120 determines whether or not the eye image acquired by the image acquisition unit 110 is an image suitable for registration (step S102). When it is determined that the eye image is an image suitable for registration (the step S102: YES), the subsequent processing is omitted and a series of operation steps is ended.

On the other hand, when it is determined that the eye image is an image unsuitable for registration (the step S102: NO), the opening detection unit 130 detects the opening degree of the eye of the target person in the eye image (step S103). In addition, in the second example embodiment, the opening degree detection unit 130 detects an eyelid overlapping the iris of the target person (step S201). For example, the opening degree detection unit 130 detects whether the eyelid overlapping the iris is an upper eyelid or a lower eyelid.

Thereafter, the guide information output unit 140 outputs the guide information based on the opening degree of the eye detected by the opening degree detection unit 130 and the eyelid overlapping the iris (step S202). Especially in the guide information according to the second example embodiment, predetermined highlighting is performed on the eyelid overlapping the iris. A highlighting aspect is not particularly limited, but may be indicated by a difference in display color, a size and thickness of characters, a movement (animation), or the like, for example.

### (Example of Guide information)

Next, with reference to FIG. 6, a specific example of the guide information in the information processing system 10 according to the second example embodiment will be described. FIG. 6 is a plan view illustrating an example of the guide information in the information processing system according to the second example embodiment.

As illustrated in FIG. 6, in the guide information outputted by the information processing system 10 according to the second example embodiment, the predetermined highlighting is performed on the eyelid overlapping the iris, as described above. Specifically, when the iris is hidden by the lower eyelid, a lower eyelid part is highlighted. In addition, when the iris is hidden by the upper eyelid, an upper eyelid part is highlighted. When the iris is hidden by both the upper eyelid and the lower eyelid, both the upper eyelid and the lower eyelid may be highlighted. Alternatively, one of the upper and lower eyelids that overlaps the iris more, may be highlighted.

The highlighting may be performed on the entire upper or lower eyelid, or only on the part that overlaps the iris. In the example illustrated in FIG. 6, a thick line in striking and showy color (e.g., red or fluorescent color, etc.) is superimposed and displayed on the part of the eyelid overlapping the iris, and it flashes at predetermined intervals. That is, it is possible to make the eyelid that causes the iris to be hidden (in other words, the eyelid that is required to be opened with the finger) stand out. Furthermore, a part other than the eyelid may be highlighted. For example, a lower half of the eye may be highlighted when the iris is hidden by the lower eyelid, and an upper half of the eye may be highlighted when the iris is hidden by the upper eyelid.

### (Technical Effect)

Next, a technical effect obtained by the information processing system 10 according to the second example embodiment will be described.

As described in FIG. 5 and FIG. 6, in the information processing system 10 according to the second example embodiment, the predetermined highlighting is performed on the eyelid overlapping the iris. In this way, it is possible to properly explain to the target person which side of the eyelids should be opened with the finger.

### <Third Example Embodiment>

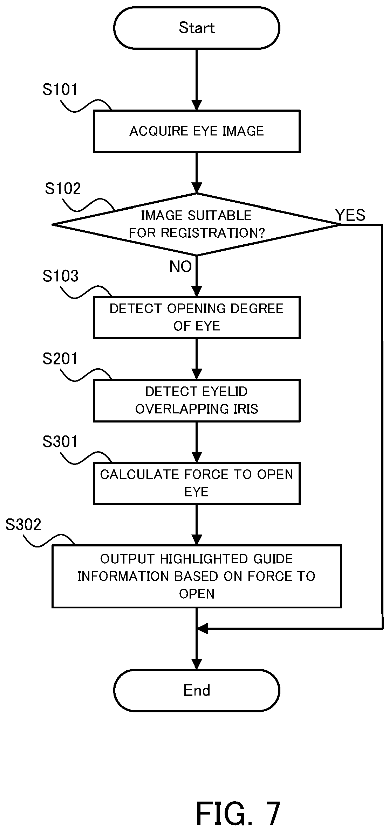

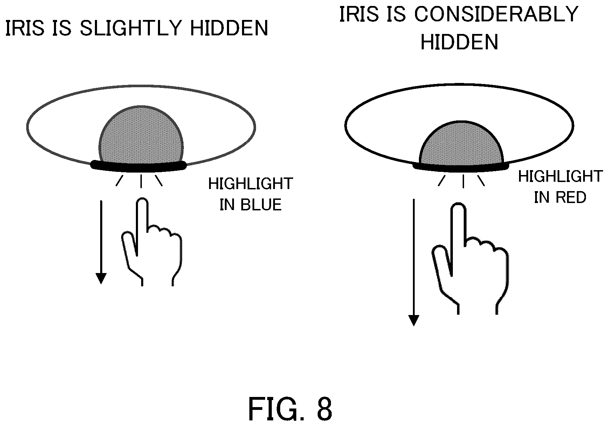

The information processing system 10 according to a third example embodiment will be described with reference to FIG. 7 and FIG. 8. The third example embodiment is partially different from the second example embodiment only in operation, and may be the same as the first and second example embodiments in the other parts. For this reason, a part that is different from each of the example embodiments described above will be described in detail below, and a description of the other overlapping parts will be omitted as appropriate.

### (Flow of Operation)

First, with reference to FIG. 7, a flow of operation by the information processing system 10 according to the third example embodiment will be described. FIG. 7 is a flowchart illustrating the flow of the operation of the information processing system according to the third example embodiment. In FIG. 7, the same steps as those illustrated in FIG. 5 carry the same reference numerals.

As illustrated in FIG. 7, when the operation of the information processing system 10 according to the third example embodiment is started, first, the image acquisition unit 110 acquires the eye image including the eye of the target person (step S101). Then, the quality determination unit 120 determines whether or not the eye image acquired by the image acquisition unit 110 is an image suitable for registration (step S102). When it is determined that the eye image is an image suitable for registration (the step S102: YES), the subsequent processing is omitted and a series of operation steps is ended.

On the other hand, when it is determined that the eye image is an image unsuitable for registration (the step S102: NO), the opening detection unit 130 detects the opening degree of the eye of the target person in the eye image (step S103). In addition, in the second example embodiment, the opening degree detection unit 130 detects an eyelid overlapping the iris of the target person (step S201).

Furthermore, in the third example embodiment, the opening detection unit 130 calculates force/strength to open the eye, from the opening of the eye (step S301). That is, the opening degree calculation unit 130 calculates how much force the target person needs to put on the finger so as to open the eye and capture an appropriate image. The force to open the eye may be calculated to be weak when the opening degree of the eye is large, and may be calculated to be strong when the opening degree of the eye is small.

Thereafter, the guide information output unit 140 outputs the guide information based on the opening degree of the eye detected by the opening degree detection unit 130, the eyelid overlapping the iris, and the force to open the eye (step S302). Especially in the guide information according to the third example embodiment, the highlighting aspect is changed depending on the force to open the eye. That is, different highlighting is performed when the force to open the eye is strong and when it is weak.

### (Example of Guide information)

Next, with reference to FIG. 8, a specific example of the guide information in the information processing system 10 according to the third example embodiment will be described. FIG. 8 is a plan view illustrating an example of the guide information in the information processing system according to the third example embodiment.

As illustrated in FIG. 8, the guide information outputted by the information processing system 10 according to the third example embodiment is highlighted in accordance with the force to open the eye. Specifically, when the iris is slightly hidden (in other words, when the force to open the eye may be weak), the highlighting is performed in blue. On the other hand, when the iris is considerably hidden (in other words, when the force to open the eye is required to be large), the highlighting is performed in red. In addition, when the iris is considerably hidden, the image of the finger and the arrow are displayed relatively larger than those when the iris is slightly hidden. The highlighting aspect described above is merely an example, and a display aspect may be changed as appropriate so as to make it easier to intuitively understand how strong the force is.

### (Technical Effect)

Next, a technical effect obtained by the information processing system 10 according to the third example embodiment will be described.

As described in FIG. 7 and FIG. 8, in the information processing system 10 according to the third example embodiment, the highlighting aspect is changed depending on the strength of the force required when the eye is opened with the finger. In this way, it is possible to properly explain to the target person how much force is required to open the eyelid.

### <Fourth Example Embodiment>

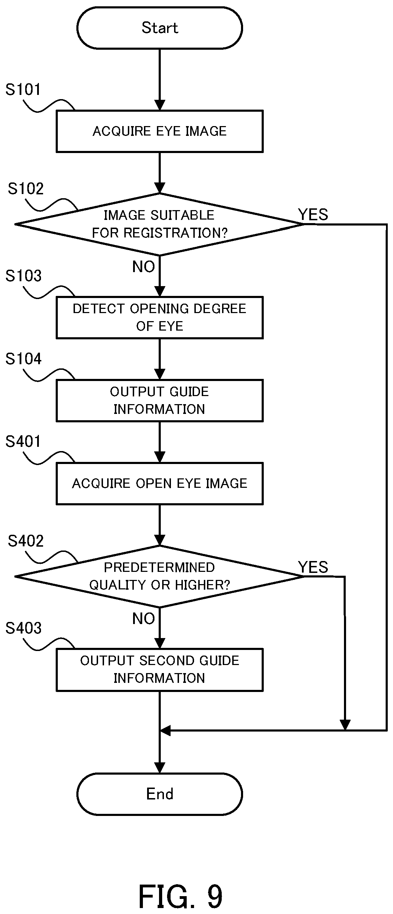

The information processing system 10 according to a fourth example embodiment will be described with reference to FIG. 9. The fourth example embodiment is partially different from the first to third example embodiments only in the operation, and may be the same as the first to third example embodiments in the other parts. For this reason, a part that is different from each of the example embodiments described above will be described in detail below, and a description of the other overlapping parts will be omitted as appropriate.

### (Flow of Operation)

First, with reference to FIG. 9, a flow of operation by the information processing system 10 according to the fourth example embodiment will be described. FIG. 9 is a flowchart illustrating the flow of the operation of the information processing system according to the fourth example embodiment. In FIG. 9, the same steps as those illustrated in FIG. 3 carry the same reference numerals.

As illustrated in FIG. 9, when the operation of the information processing system 10 according to the fourth example embodiment is started, first, the image acquisition unit 110 acquires the eye image including the eye of the target person (step S101). Then, the quality determination unit 120 determines whether or not the eye image acquired by the image acquisition unit 110 is an image suitable for registration (step S102). When it is determined that the eye image is an image suitable for registration (the step S102: YES), the subsequent processing is omitted and a series of operation steps is ended.

On the other hand, when it is determined that the eye image is an image unsuitable for registration (the step S102: NO), the opening detection unit 130 detects the opening degree of the eye of the target person in the eye image (step S103). Then, the guide information output unit 140 outputs the guide information based on the opening degree of the eye detected by the opening degree detection unit 130 (step S104).

Thereafter, especially in the fourth example embodiment, the image acquisition unit 110 acquires an image captured by the target person opening the eye with the finger (hereinafter referred to as an "open eye image") (step S401). Then, the quality determination unit 120 determines whether or not quality of the open eye image (more specifically, quality of the iris in the open eye image) is greater than or equal to predetermined quality (step S402). Determination criteria here may be the same as or different from those for determining whether or not the image is suitable for registration (i.e., the determination criteria in the step S102). For example, the determination criteria in the step S402 may be a little more severe than those in the step S102, or conversely, the determination criteria may be a little lenient.

When it is determined that the quality of the open eye image is greater than or equal to the predetermined quality (the step S402: YES), the subsequent processing is omitted and a series of operation steps is ended. At this time, the information processing system10 may perform processing of recording the open eye image as the eye image of the target person.

On the other hand, when it is determined that the quality of the open eye image is less than the predetermined quality (the step S402: NO), the guide information output unit 140 further outputs second guide information for improving the quality of the open eye image (step S403). The second guide information may be the same as the firstly outputted guide information (i.e., the guide information outputted in the step S104). Alternatively, the second guide information may be different from the firstly outputted guide information. For example, the second guide information may include information for finely adjusting an action of opening the eye by the target person that is already done, such as "Please lift the eyelid with a little stronger force." In addition, the second guide information may include information other than the information for encouraging the target person to open the eye with the finger. For example, the second guidance information may include a message such as "Please bring the eye a little closer to the camera."

### (Technical Effect)

Next, a technical effect obtained by the information processing system 10 according to the fourth example embodiment will be described.

As illustrated in FIG. 9, in the information processing system 10 according to the fourth example embodiment, when the quality of the image after the output of the guide information is less than the predetermined quality, the second guide information is further outputted. In this way, even when the eye is not sufficiently opened by the firstly outputted guide information, it is possible to encourage an action for further improving the quality of the eye image by using the second guide information.

### <Fifth Example Embodiment>

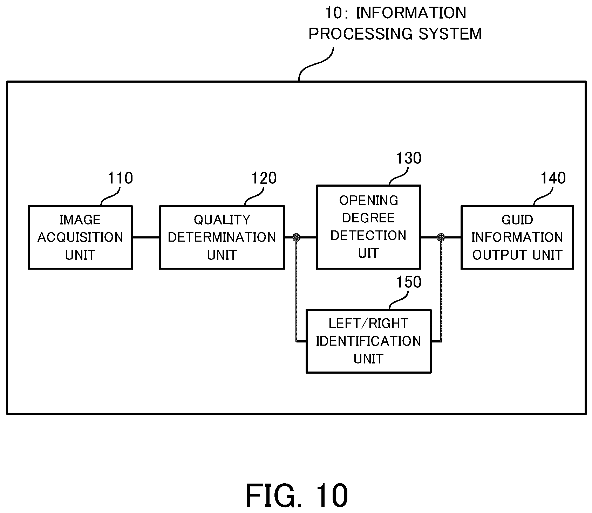

The information processing system 10 according to a fifth example embodiment will be described with reference to FIG. 10 to FIG. 12. The fifth example embodiment is partially different the first to fourth example embodiments only in the configuration and operation, and may be the same as the first to fourth example embodiments in the other parts. For this reason, a part that is different from each of the example embodiments described above will be described in detail below, and a description of the other overlapping parts will be omitted as appropriate.

### (Functional Configuration)

First, with reference to FIG. 10, a functional configuration of the information processing system 10 according to the fifth example embodiment will be described. FIG. 10 is a block diagram illustrating the functional configuration of the information processing system according to the fifth example embodiment. In FIG. 10, the same components as those illustrated in FIG. 2 carry the same reference numerals.

As illustrated in FIG. 10, the information processing system 10 according to the fifth example embodiment includes, as components for realizing the functions thereof, the image acquisition unit 110, the quality determination unit 120, the opening degree detection unit 130, the guide information output unit 140, and a left/right identification unit 150. That is, the information processing system 10 according to the fifth example embodiment further includes the left/right identification unit 150 in addition to the configuration in the first example embodiment (see FIG. 2). The left/right identification unit 150 may be a processing block realized or implemented by the processor 11 (see FIG. 1), for example.

The left/right identification unit 150 is configured to identify whether the target person holds a terminal for capturing the eye image (e.g., a smartphone equipped with a camera) with a right hand or a left hand. The left/right identification unit 150 may determine with which hand the target person holds the terminal, on the basis of outputs of various sensors mounted on the terminal (e.g., an inclination sensor or a gyro sensor, etc.), for example. Alternatively, the left/right identification unit 150 may determine with which hand the target person holds the terminal, on the basis of the eye image acquired by the image acquisition unit 110. More specifically, the left/right identification unit 150 may determine with which hand the target person holds the terminal, from an angle of view of the eye image or the like. An identification result by the left/right identification unit 150 is configured to be outputted to the guide information output unit 140.

The guide information output unit 140 according to the fifth example embodiment is configured to output the guide information based on the identification result of the left/right identification unit 150 (i.e., whether the target person holds the terminal with the right hand or the left hand), in addition to the opening degree of the eye detected by the opening degree detection unit 140. A specific example of the guide information will be described in detail later.

### (Flow of Operation)

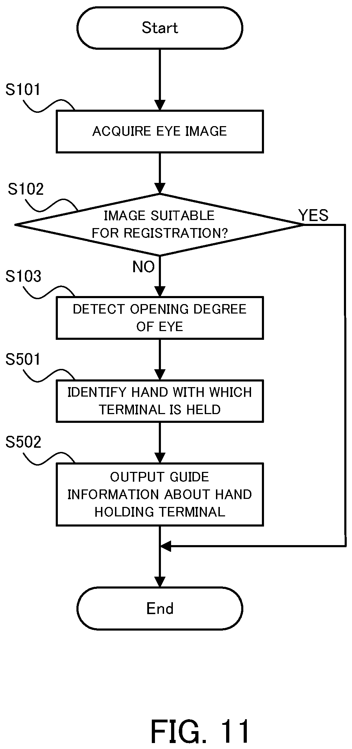

Next, with reference to FIG. 11, a flow of operation by the information processing system 10 according to the fifth example embodiment will be described. FIG. 11 is a flowchart illustrating the flow of the operation of the information processing system according to the fifth example embodiment. In FIG. 11, the same steps as those illustrated in FIG. 3 carry the same reference numerals.

As illustrated in FIG. 11, when the operation of the information processing system 10 according to the fifth example embodiment is started, first, the image acquisition unit 110 acquires the eye image including the eye of the target person (step S101). Then, the quality determination unit 120 determines whether or not the eye image acquired by the image acquisition unit 110 is an image suitable for registration (step S102). When it is determined that the eye image is an image suitable for registration (the step S102: YES), the subsequent processing is omitted and a series of operation steps is ended.

On the other hand, when it is determined that the eye image is an image unsuitable for registration (the step S102: NO), the opening detection unit 130 detects the opening degree of the eye of the target person in the eye image (step S103). Furthermore, in the fifth example embodiment, the left/right identification unit 150 identifies whether the target person holds the terminal with the left hand or the right hand (step S501).

Thereafter, the guide information output unit 140 outputs the guide information based on the opening degree of the eye detected by the opening degree detection unit 130 and information about the hand holding the terminal identified by the left/right identification unit 150 (step S502). Especially, the content of the guide information according to the fifth example embodiment varies depending on whether the target person holds the terminal with the right hand or the left hand.

### (Example of Guide information)

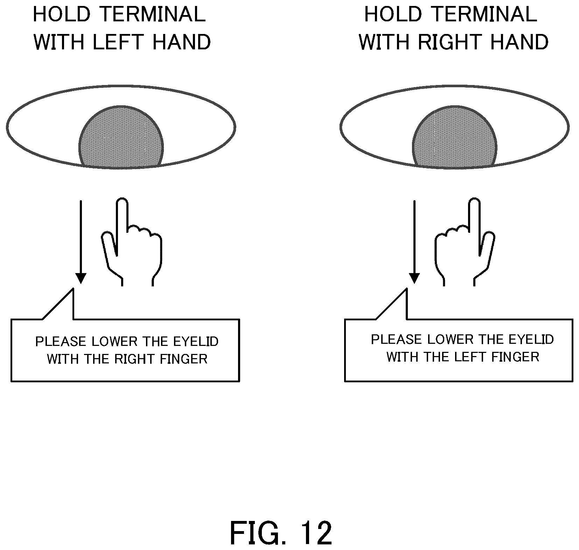

Next, with reference to FIG. 12, a specific example of the guide information in the information processing system 10 according to the fifth example embodiment will be described. FIG. 12 is a plan view illustrating an example of the guide information in the information processing system according to the fifth example embodiment.

As illustrated in FIG. 12, in the information processing system 10 according to the fifth example embodiment, when the target person holds the terminal with the left hand, the guide information for opening the eye with a right finger is outputted. For example, the guide information may include a message for specifying the hand to use, such as "Please lower the eyelid with the right finger." Furthermore, an image of the right hand may be displayed to indicate the hand to use.

On the other hand, when the target person holds the terminal with the right hand, the guide information for opening the eye with a left finger is outputted. For example, the guide information may include a message for specifying the hand to use, such as "Please lower the eyelid with the left finger." Furthermore, an image of the left hand may be displayed to indicate the hand to use.

As described in FIG. 10 to FIG. 12, in the information processing system 10 according to the fifth example embodiment, the guide information for opening the eye with the finger of the hand that is opposite to the hand with which the target person holds the terminal. In this way, it is possible to encourage the target person who holds the terminal, to open the eye in an easier-to-understand manner.

A processing method that is executed on a computer by recording, on a recording medium, a program for allowing the configuration in each of the example embodiments to be operated so as to realize the functions in each example embodiment, and by reading, as a code, the program recorded on the recording medium, is also included in the scope of each of the example embodiments. That is, a computer-readable recording medium is also included in the range of each of the example embodiments. Not only the recording medium on which the above-described program is recorded, but also the program itself is also included in each example embodiment.

The recording medium to use may be, for example, a floppy disk (registered trademark), a hard disk, an optical disk, a magneto-optical disk, a CD-ROM, a magnetic tape, a nonvolatile memory card, or a ROM. Furthermore, not only the program that is recorded on the recording medium and that executes processing alone, but also the program that operates on an OS and that executes processing in cooperation with the functions of expansion boards and another software, is also included in the scope of each of the example embodiments. In addition, the program itself may be stored in a server, and a part or all of the program may be downloaded from the server to a user terminal. The program may be provided to a user in a form of SaaS (Software as a Service), for example.

### <Supplementary Notes>

The example embodiments described above may be further described as, but not limited to, the following Supplementary Notes below.

### (Supplementary Note 1)

An information processing system according to Supplementary Note 1 is an information processing system including: an acquisition unit that acquires an eye image including an eye of a target person; a determination unit that determines whether or not the eye image is an image suitable for registration; a detection unit that detects an opening degree of the eye of the target person in the eye image in a case where it is determined that the eye image is not suitable for registration; and an output unit that outputs guide information for encouraging the target person to open the eye with a finger, based on the opening degree of the eye.

### (Supplementary Note 2)

An information processing system according to Supplementary Note 2 is the information processing system according to Supplementary Note 1, wherein the detection unit detects an eyelid overlapping an iris of the target person, and the output unit outputs the guide information in which predetermined highlighting is performed on the eyelid overlapping the iris.

### (Supplementary Note 3)

An information processing system according to Supplementary Note 3 is the information processing system according to Supplementary Note 2, wherein the output unit changes an aspect of the predetermined highlighting depending on strength of a force required to open the eye with the finger.

### (Supplementary Note 4)

An information processing system according to Supplementary Note 4 is the information processing system according to any one of Supplementary Notes 1 to 3, wherein the acquisition unit acquires an open eye image including the eye opened with the finger after the guide information is outputted, the determination unit determines whether or not quality of an iris of the target person in the open eye image is greater than or equal to predetermined quality, and the output unit further outputs second guide information for improving the quality of the iris, in a case where the quality of the iris is less than the predetermined quality.

### (Supplementary Note 5)

An information processing system according to Supplementary Note 5 is the information processing system according to any one of Supplementary Notes 1 to 3, further including an identification unit that identifies with which of a right hand and a left hand the target person holds a terminal for capturing the eye image, wherein the output unit outputs the guide information for opening the eye with a finger of a hand that is opposite to a hand holding the terminal.

### (Supplementary Note 6)

An information processing method according to Supplementary Note 6 is an information processing method that is executed by at least one computer, the information processing method including: acquiring an eye image including an eye of a target person; determining whether or not the eye image is an image suitable for registration; detecting an opening degree of the eye of the target person in the eye image in a case where it is determined that the eye image is not suitable for registration; and outputting guide information for encouraging the target person to open the eye with a finger, based on the opening degree of the eye.

### (Supplementary Note 7)

A recording medium according to Supplementary Note 7 is a recording medium on which a computer program that allows at least one computer to execute an information processing method is recorded, the information processing method including: acquiring an eye image including an eye of a target person; determining whether or not the eye image is an image suitable for registration; detecting an opening degree of the eye of the target person in the eye image in a case where it is determined that the eye image is not suitable for registration; and outputting guide information for encouraging the target person to open the eye with a finger, based on the opening degree of the eye.

### (Supplementary Note 8)

A computer program according to Supplementary Note 8 is a computer program that allows at least one computer to execute an information processing method, the information processing method including: acquiring an eye image including an eye of a target person; determining whether or not the eye image is an image suitable for registration; detecting an opening degree of the eye of the target person in the eye image in a case where it is determined that the eye image is not suitable for registration; and outputting guide information for encouraging the target person to open the eye with a finger, based on the opening degree of the eye.

This disclosure is allowed to be changed, if desired, without departing from the essence or spirit of this disclosure which can be read from the claims and the entire specification. An information processing system, an information processing method, and a recording medium with such changes are also intended to be within the technical scope of this disclosure.

### Description of Reference Codes

10 Information processing system
11 Processor
110 Image acquisition unit
120 Quality determination unit
130 Opening degree detection unit
140 Guide information output unit
150 Left and right identification unit

## Claims

1. An information processing system comprising:
an acquisition unit that acquires an eye image including an eye of a target person;
a determination unit that determines whether or not the eye image is an image suitable for registration;
a detection unit that detects an opening degree of the eye of the target person in the eye image in a case where it is determined that the eye image is not suitable for registration; and
an output unit that outputs guide information for encouraging the target person to open the eye with a finger, based on the opening degree of the eye.

2. The information processing system according to claim 1, wherein
the detection unit detects an eyelid overlapping an iris of the target person, and
the output unit outputs the guide information in which predetermined highlighting is performed on the eyelid overlapping the iris.

3. The information processing system according to claim 2, wherein the output unit changes an aspect of the predetermined highlighting depending on strength of a force required to open the eye with the finger.

4. The information processing system according to any one of claims 1 to 3, wherein
the acquisition unit acquires an open eye image including the eye opened with the finger after the guide information is outputted,
the determination unit determines whether or not quality of an iris of the target person in the open eye image is greater than or equal to predetermined quality, and
the output unit further outputs second guide information for improving the quality of the iris, in a case where the quality of the iris is less than the predetermined quality.

5. The information processing system according to any one of claims 1 to 3, further comprising an identification unit that identifies with which of a right hand and a left hand the target person holds a terminal for capturing the eye image, wherein
the output unit outputs the guide information for opening the eye with a finger of a hand that is opposite to a hand holding the terminal.

6. An information processing method that is executed by at least one computer, the information processing method comprising:
acquiring an eye image including an eye of a target person;
determining whether or not the eye image is an image suitable for registration;
detecting an opening degree of the eye of the target person in the eye image in a case where it is determined that the eye image is not suitable for registration; and
outputting guide information for encouraging the target person to open the eye with a finger, based on the opening degree of the eye.

7. A recording medium on which a computer program that allows at least one computer to execute an information processing method is recorded, the information processing method including:
acquiring an eye image including an eye of a target person;
determining whether or not the eye image is an image suitable for registration;
detecting an opening degree of the eye of the target person in the eye image in a case where it is determined that the eye image is not suitable for registration; and
outputting guide information for encouraging the target person to open the eye with a finger, based on the opening degree of the eye.
